# EUROPÄISCHE PATENTSCHRIFT

(11) **EP 3 768 109 B1**
(45) Veröffentlichungstag und Bekanntmachung des Hinweises auf die Patenterteilung: **04.06.2025**
(21) Anmeldenummer: 19714314.2
(22) Anmeldetag: 18.03.2019
(51) Int. Cl.: A43D 1/02, A43B 7/1464

(54) **VERFAHREN, VORRICHTUNG UND SYSTEM ZUM MESSEN, BEURTEILEN UND ZUR SIMULATION EINES SCHUHS**
METHOD, DEVICE AND SYSTEM FOR MEASURING, EVALUATING AND SIMULATING A SHOE
PROCÉDÉ, DISPOSITIF ET SYSTÈME DE MESURE, D'ÉVALUATION ET DE SIMULATION D'UNE CHAUSSURE

(30) Priorität: 20.03.2018 DE 102018002283; 20.03.2018 DE 102018002284
(43) Veröffentlichungstag der Anmeldung: 27.01.2021
(73) Patentinhaber: molibso Entwicklungs- und Vertriebs GmbH, 40764 Langenfeld (DE)
(72) Erfinder: HOLLENBACHER, Jens, 40764 Langenfeld (DE)
(74) Vertreter: Cohausz Hannig Borkowski Wißgott
(86) Internationale Anmeldenummer: PCT/EP2019/000083
(87) Internationale Veröffentlichungsnummer: WO 2019/179655

(56) Entgegenhaltungen:
- WO-A1-2018/048880
- KR-A- 20070 100 195
- US-A1- 2012 198 949
- US-A1- 2013 219 745
- US-A1- 2016 135 537
- US-A1- 2016 345 667
- US-A1- 2017 164 899

## Beschreibung

Die Erfindung betrifft ein Verfahren zum Messen und Beurteilen der Funktionen und Form eines Schuhs insbesondere eines Sportschuhs wie eines Lauf- oder Skischuhs. Auch betrifft die Erfindung ein Verfahren zur Generierung von Daten für die Herstellung eines individuell an eine Person angepassten Schuhs. Des Weiteren betrifft die Erfindung ein System zur Simulation eines Schuhs, das zur Generierung von Daten für die Herstellung von Schuhen verwendbar ist. Zudem betrifft die Erfindung eine Schuhsimulationsvorrichtung die das System aufweist und mittels der das Verfahren durchführbar ist.

Es ist bekannt, innerhalb eines Schuhs Drucksensoren anzuordnen, um die von der Fußsohle erzeugten unterschiedlichen Drücke zu erfassen. Hierbei befinden sich die Drucksensoren entweder in der Oberseite des Schuhbodens oder in einer Schuheinlage. Um zu den gewünschten Messwerten zu kommen, ist es erforderlich, Schuhe oder Schuheinlagen mit Sensoren zu versehen. Dies ist nicht nur aufwändig in der Herstellung, sondern verändert auch die Form und Abmessungen des Schuhs derart, dass verwertbare Messergebnisse kaum zu erreichen sind.

Bei der Herstellung von Schuhen kommen zunehmend Verfahren zum Einsatz, die es ermöglichen einzelne Bestandteile der Schuhe individuell an eine Person anzupassen und somit die Passform der resultierenden Schuhe zu verbessern. Insbesondere können Formteile durch 3D-Drucktechniken passgenau gestaltet werden.

Es ist bekannt bestimmte Teile von Schuhen auf Grundlage von Messwerten individuell an die Bedürfnisse einer Person anzupassen. Beispielsweise ist aus der DE 102016 009 980 A1 bekannt ein aus Schaum geformtes Formteil, insbesondere den Schuhboden eines Sportschuhs, an eine Person anzupassen, indem eine Druckverteilung zumindest eines Fußes einer Person mittels einer Druckmessplatte gemessen wird und auf Grundlage der bestimmten Druckverteilung die Materialeigenschaften des Schaumaterials räumlich eingestellt werden. US 2016/135537 A1 bezieht sich auf Systeme und Verfahren zur Gestaltung und Herstellung von individualisierten Schuhen.

Bei der Fertigung von Teilen auf alleiniger Grundlage von Messwerten hat die Person erst die Möglichkeit zu erfahren wie sich der Schuh anfühlt, wenn dieser bereits gefertigt worden ist. Insbesondere hat die Person keinen unmittelbaren Einfluss auf das Endprodukt. In Folge dessen kann es vorkommen, dass die Person mit dem hergestellten Schuh nicht zufrieden ist, da dieser gegenüber der Wunschvorstellung der Person Unterschiede aufweist.

Aufgabe der vorliegenden Erfindung ist es, ein möglichst einfaches und praktikables Verfahren und System zu Verfügung zu stellen, dass in einem Ladengeschäft einsetzbar ist und es ermöglicht Daten zu generieren, an Hand derer ein Schuh herstellbar ist, der den Wünschen der Person entspricht.

Auch ist es Aufgabe der Erfindung, ein Verfahren zum Messen und Beurteilen der Funktionen und Form eines Schuhs insbesondere eines Sportschuhs zu schaffen, durch das Messwerte von Schuhen insbesondere Sportschuhen erreicht werden, ohne die Schuhe/Sportschuhe verändern zu müssen.

Die Erfindung ist im beigefügten Anspruchssatz beschrieben.

Nach den erfassten Druckwerten werden die Form, die Abmessungen und/oder die Materialien des Schuhbodens bestimmt.

Ferner können nach den erfassten Druckwerten die Form und die Abmessungen des Schuhschaftes bestimmt werden.

Damit kann jeder Schuh vermessen, beurteilt und optimal angefertigt werden, ohne ihn mit Sensoren versehen zu müssen. Folgende Eigenschaften können bei jeder Schuhart erkannt und berücksichtigt werden:
- Passt der Schuh zur Form des jeweiligen Fußes?
- Passt der Schuh zu der individuellen Bewegung des Benutzers?
- Geht/Läuft der Benutzer fehlerhaft?
- Welche Form und Abmessungen sollte der Schuhboden insbesondere dessen Lauffläche und/oder dessen Oberseite haben?
- Welche mechanischen Eigenschaften sollten der Schuhboden und/oder der Schuhschaft haben?
- Welche Materialien sollten für den Schuhboden und/oder für den Schuhschaft verwendet werden?
- Wie ist das Verschleißverhalten des Schuhs?

Beim Messschritt wird zunächst die Druckverteilung Schuhs mittels einer Druckverteilungsmessvorrichtung bestimmt, wobei die Druckverteilung statisch, d.h. stehend, oder dynamisch, d.h. gehend oder laufend, gemessen werden kann.

Auf Grundlage der bestimmten Druckverteilung wird anschließend die Schuhsimulationsvorrichtung eingestellt. Um die Eigenschaften eines Schuhs wahrzunehmen, der die eingestellten Eigenschaften aufweist, benutzt die Person die Schuhsimulationsvorrichtung, in dem sie den Schuhboden oder die Schuheinlage belastet. Die Belastung der Schuhsimulationsvorrichtung kann statisch, d.h. stehend, oder dynamisch, d.h. gehend oder laufend, erfolgen. Mit Hilfe der Schuhsimulationsvorrichtung nimmt die Person den Schuhboden mit den Eigenschaften wahr, die ein Schuh nach der Herstellung aufweist. So lässt sich vorteilhafterweise erreichen, dass die Person beim Kauf des Schuhs die Eigenschaften kennt und somit Fehlkäufe vermieden werden.

Zur Simulation eines Schuhs weist die Schuhsimulationsvorrichtung einen Schuhboden auf, der wiederum eine Vielzahl von Auflageabschnitten aufweist, wobei die Auflageabschnitte einzeln in Höhe und zumindest einer physikalischen Eigenschaft steuerbar sind. Durch Änderung der Höhe der einzelnen Auflageabschnitte ist das Profil des Schuhbodens individuell einstellbar. Anhand der Druckverteilung kann beispielsweise bestimmt werden, welche Anschnitte des Schuhbodens von der Person stärker belastet werden, so dass für diese Abschnitte eine geringere Höhe eingestellt wird. Die Auflageabschnitte sind dafür vorgesehen, dass die mechanischen Eigenschaften für jeden der Auflageabschnitte jeweils einzeln und unabhängig voneinander einstellbar sind. Auf diese Weise lässt sich ein Schuhboden simulieren, der gegenüber dem hergestellten Schuh vergleichbare mechanische Eigenschaften aufweist.

Die Schuhsimulationsvorrichtung kann alternativ auch eine Schuheinlage aufweisen, die im bestimmungsgemäßen Gebrauch in einen Schuh eines bestimmten Typs eingelegt wird und die eine Vielzahl von einstellbaren Auflageabschnitten aufweist. Auf diese Weise lässt sich eine individuell angepasste Schuheinlage für einen bestimmten Schuh herstellen. Mit einer individuell angepassten Schuheinlage als Bestandteil eines Schuhs lässt sich ein individuell angepasster Schuh realisieren.

Die steuerbaren mechanischen Eigenschaften der Auflageabschnitte sind bei dem Schuhboden und bei der Schuheinlage Härte, Flexibilität und Elastizität. Besonders bevorzugt ist die Steuerung der Härte.

Eine Ausführung des Verfahrens zeichnet sich dadurch aus, dass dieses einen zusätzlichen Begutachtungsschritt aufweist, bei dem von der Person begutachtet wird, ob die lokal eingestellten Werte der Schuhsimulationsvorrichtung dem persönlichen Optimum oder Wunsch entsprechen. Sollte die Wunscheinstellung durch die aktuelle eingestellten Werte für die Höhe und/oder die mechanischen Eigenschaften der Auflageabschnitte nicht vorliegen, wird ein weiterer Einstellschritt mit geänderten eingestellten Werten durchgeführt. Ob die Einstellungen dem persönlichen Optimum entsprechen, kann die Person und/oder ein Dritter, beispielsweise ein Mitarbeiter des Ladengeschäftes, an der Rechnereinheit mittels einer Bedieneinheit eingeben. Vorzugsweise lässt sich dafür eine mobile Eingabevorrichtung verwenden, die über Funk mit der Rechnereinheit verbunden ist. Die Berechnung neuer geänderter Werte für die Höhe und/oder die mechanischen Eigenschaften wird mittels der Rechnereinheit vorgenommen, wobei für im Wesentlichen alle Auflageabschnitte neu einzustellende Werte berechnet werden, die die vorliegenden eingestellten Werte ersetzen. Bevorzugt können voreingestellte Parameter ausgewählt werden, mittels derer die Person zwischen einer insgesamt weicheren oder härteren Einstellung der Auflageabschnitte wählen kann. Die Auswahl kann vorzugsweise durch die Eingabevorrichtung durchgeführt werden.

Die Schuhsimulationsvorrichtung ist vorzugsweise so ausgeführt, dass die Person diese beim Einstellschritt benutzt, d.h. den Schuhboden belastet. Der Vorteil der Vornahme des Einstellschrittes beim belasteten Schuhboden besteht darin, dass die Person die Änderung unmittelbar wahrnimmt und so besser einordnen kann, ob eine Veränderung in die gewünschte Richtung stattfindet.

Herstellung, Messung und Beurteilung werden noch wesentlich verbessert, wenn die Daten/Messdaten eines bekannten optimalen Schuhs erfasst werden und diese Daten mit den durch eines der obigen Verfahren gemessenen Daten verglichen und verwertet werden.

Bei einer Ausführung des Verfahren wird ein Optimierungsschritt durchgeführt, bei dem die Höhe und/oder die zumindest eine mechanische Eigenschaft der Auflageabschnitte mittels einer Eingabevorrichtung jeweils lokal eingestellt werden. Auf diese Weise wird der Person, oder einem Dritter, beispielsweise einem Mitarbeiter des Ladengeschäftes, ermöglich mittels einer Eingabevorrichtung lokale Einstellung der Werte der einzelnen Auflageabschnitte vorzunehmen und so den Schuhboden individuelle zu modifizieren. Die Eingabevorrichtung kann ein mobiles Endgerät mit einer Programmanwendung sein, welches Daten mit der Rechnereinheit über Funk austauscht. Auch beim Optimierungsschritt kann die Schuhsimulationsvorrichtung benutzt sein, d.h. der Schuhboden belastet sein, oder nicht benutz sein, d.h. der Schuboden entlastet sein. Die einstellbare lokale Veränderung der Eigenschaften der Auflageabschnitte ist insbesondere vorteilhaft für Personen, bei denen aus orthopädischen oder medizinischen Gründen eine lokale Änderung des Höhenprofils und/oder der mechanischen Eigenschaften erforderlich ist. Dies kann beispielsweise bei Menschen mit neurologischen Störungen, wie z.B. bei Diabetes, von Vorteil sein, um bestimmte Reize an der Fußsohle zu setzen um die eigene Wahrnehmung der Körperbewegung (Propriozeption) zu verbessern.

Vorzugsweise wird bei dem Verfahren zumindest ein Haltemittel eingesetzt, das bei der Simulationsvorrichtung dafür vorgesehen ist den Fuß der Person auf dem Schuhboden zu fixieren, wobei dieses in seiner Länge verändert wird, um an die Anatomie der jeweiligen Person angepasst zu werden. Damit lässt sich durch die Schuhsimulationsvorrichtung zusätzlich zum Schuhboden oder der Schuheinlage auch der Schuhoberbau simulieren, so dass die Person den simulierten Schuh als zusammenhängende Einheit wahrnimmt. Die genaue Gestaltung der Haltmittel kann je nach Schuhtyp unterschiedlich sein. Vorzugsweise verfügt zumindest eines der Haltemittel über eine Kontraktionseinheit, dessen Länge mittels der Rechnereinheit, der Eingabevorrichtung und/oder manuell auf einen gewünschten Wert einstellbar ist. Auf diese Weise kann die Länge der jeweiligen Haltemittel gezielt gesteuert und für die jeweilige Person gezielt festgehalten werden. Die Steuerung der Länge der Kontraktionseinheit mittels der Eingabevorrichtung hat den Vorteil, dass die Person die Länge der Haltemittel gezielt steuern kann, und zwar bevorzugt, wenn sie die Schuhsimulationsvorrichtung benutzt, also den Schuboden oder die Schuheinlage belastet. Dadurch lässt sich für die Person unmittelbar erfahren, welche Auswirkung die Änderung der Längen einzelner Haltemittel hat. Insbesondere lassen sich auf diese Weise in Kombination mit der lokalen Änderung der Werte für die Höhe und/oder die mechanischen Eigenschaften der Auflageabschnitte individuell optimierte Einstellungen finden, um dem Wunsch der Person zu entsprechen. Vorteilhafterweise lässt sich durch gezielte Einstellung der Längen der Haltemittel ein orthopädie-technischer Effekt erzielen.

Eine Ausführung des Verfahrens zeichnet sich dadurch aus, dass die von der Person als optimal angesehenen Werte für die Höhe und/oder die zumindest eine mechanische Eigenschaft der Auflageabschnitte und/oder die Länge der Haltemittel in einer Speichereinheit abgespeichert werden. Auf diese Weise lassen sich die optimalen Einstellungen für eine Person festhalten und an beliebigen Orten zu beliebigen Zeiten reproduzieren. Beispielsweise kann eine anderswo lokalisierte Schuhsimulationsvorrichtung mit den abgespeicherten Werte angesteuert werden, so dass die Einstellung für eine Person an einem anderen Ort erfahrbar ist. Zudem kann die Person den simulierten Schuh zu einem späteren Zeitpunkt wahrnehmen, beispielsweise wenn die Person sich erst später dazu entschließt einen Kauf oder eine Kaufentscheidung vorzunehmen.

Bei einer weiteren Ausführung der Erfindung werden die abgespeicherten Werte als Daten für die Herstellung von individuell angepassten Schuhen verwendet. Durch die als Werte für Höhe und/oder die zumindest eine mechanische Eigenschaft der Auflageabschnitte und/oder die Länge der Haltemittel abgespeicherten Daten ist es möglich den als optimal angesehenen Schuh an einem anderen Ort zu produzieren. Zwischen dem produzierten Schuh und dem simulierten Schuh gibt es so bis auf Fertigungstoleranzen keinen Unterschied mehr. Vorteilhafterweise lassen sich so Fehlkäufe vermeiden.

Vorzugsweise wird beim zugrundeliegenden Verfahren im Rahmen des Messschrittes zusätzlich die räumliche Form des Fußes der Person mittels mindestens einer Scanvorrichtung bestimmt. Durch die Verwendung einer Scanvorrichtung erhält man zusätzliche Informationen, die unmittelbar für die Bestimmung der Höhen der Auflageabschnitte, respektive für das Profil des Schuhbodens oder der Schuheinlage, verwendbar sind. Als Scanvorrichtungen können handelsübliche 3D-Scanner verwendet werden. Vorzugsweise wird die Form des Fußes vermessen, wenn dieser sich frei im Raum befindet, also nicht belastet ist. Bei einer bevorzugten Ausführung des Verfahrens wird der Person beim Benutzen der Schuhsimulationsvorrichtung mittels einer Anzeigeeinheit optisch suggeriert, dass sie einen Schuh trägt, der eine äußere Form (Design, Farbe, Symbolik) aufweist, die die Person auswählt oder ausgewählt hat. Als Anzeigeeinheit kann ein Bildschirm verwendet werden, den die Person anschaut und bei dem ein bestimmter Schuh am Ort der Füße eingeblendet wird. Vorzugsweise ist die Anzeigeeinheit eine Virtual-Reality-Brille, bei der die Schuhe an dem Ort eingeblendet werden, an denen sich die Füße der Person befinden. Aus diese Weise wird die Immersion, d.h. die Wahrnehmung der simulierten Umgebung als Realität, verbessert und eine Wahrnehmung des simulierten Schuhs als realer Schuh erzielt. Vorzugsweise lässt sich eine äußere Erscheinungsform des Schuhs anzeigen, die der Kunde selbst entworfen hat oder entwirft, so dass auch bezüglich der äußeren Form eine Individualisierung durchführbar und wahrnehmbar ist.

Die Erfindung betrifft zudem ein System zur Simulation eines Schuhs bei der Herstellung eines individuell an eine Person angepassten Schuhs, umfassend
- eine Druckverteilungsmessvorrichtung, die dafür vorgesehen ist eine Druckverteilung zumindest eines Schuhs zu bestimmen,
- eine Rechnereinheit, die dafür vorgesehen ist die bestimmte Druckverteilung auszuwerten, und
- eine Schuhsimulationsvorrichtung, die einen Schuhboden oder eine Schuheinlage mit einer Vielzahl von Auflageabschnitten aufweist, deren Höhe und/oder zumindest eine mechanische Eigenschaft auf Grundlage der bestimmten Druckverteilung mittels der Rechnereinheit jeweils einzeln steuerbar sind.

Ein derartiges System kann dafür verwendet werden das oben beschriebene Verfahren umzusetzen und somit bei der der Herstellung eines individuell an eine Person angepassten Schuhs verwendet werden. Das System verfügt über Komponenten, die eine geringe Stellfläche aufweisen, so dass dieses System vorteilhafterweise in Ladengeschäften einsetzbar ist.

Vorzugsweise ist die Druckverteilungsmessvorrichtung des Systems eine Druckmessplatte, eine Druckmessfolie oder eine sensitive Einlegesohle. Bevorzugt ist eine Druckmessplatte, da diese eine Ortsauflösung und Drucksensitivität aufweist, die es ermöglicht die Druckverteilung der Fußsohle sowohl statisch, also im Stand, sowie dynamisch, also beim Gehen oder Laufen, zu bestimmen. Besonders bevorzugt sind kapazitive Druckmessplatten.

Eine Ausführung der Erfindung zeichnet sich dadurch aus, dass das System zumindest eine Scanvorrichtung aufweist, die dafür vorgesehen ist, die räumliche Form des Fußes zu bestimmen. Durch die Verwendung von Scansystemen lässt sich die Form des Fußes auf einfache Weise bestimmen. Die auf diese Weise gewonnene Daten können unmittelbar zur Bestimmung der Werte für die Höhen der Auflageabschnitte, respektive für das Profil des Schuhbodens, verwendet werden.

Vorzugsweise weist das System eine Eingabevorrichtung auf, die dafür vorgesehen ist die Höhe und/oder zumindest eine mechanische Eigenschaft der Auflageabschnitte jeweils ortsabhängig einzustellen. Auf diese Weise lassen sich die Werte lokal von der Person oder einem Dritten ändern und optimieren. Zudem ist die Eingabevorrichtung in einer bevorzugten Ausführung dafür vorgesehen, eine Parameterauswahl beim Einstellungsschritt vorzunehmen.

In einer Ausführung der Erfindung weist das System eine Anzeigeeinheit auf, die dafür vorgesehen ist, bei dem Benutzer den Eindruck zu erwecken, dass dieser einen Schuh eines bestimmten Typs oder Designs trägt. Das Design kann vorzugsweise durch die Person ausgewählt werden, so dass auch bezüglich der äußeren Gestaltung eine wunschgemäße Ausführung des Schuhs simulierbar ist. Vorzugsweise kann die Person die äußere Erscheinungsform selber gestalten.

Das System weist bevorzugt eine beweglich gelagerte Schuhsimulationsvorrichtung auf. Dadurch ist es möglich, dass die Schuhsimulationsvorrichtung bei einer natürlichen Gehbewegung oder Gewichtsverlagerung am entsprechenden Fuß nachgeführt wird. Vorteilhafterweise wird so ein realistisches Gefühl erzeugt. Besonders bevorzugt ist die Simulationsvorrichtung mobil ausgeführt, so dass die Person mit dieser herumgehen oder herumlaufen kann. In diesem Fall ist es bevorzugt, dass die Schuhsimulationsvorrichtung mittels Funk mit der Rechnereinheit verbunden ist.

In einer bevorzugten Ausführung der Erfindung ist die Schuhsimulationsvorrichtung vom System trennbar, wobei die eingestellten Werte für die Höhe und/oder die zumindest eine mechanische Eigenschaft beibehalten bleiben. Dies ist besonders bevorzugt, wenn die Schuhsimulationsvorrichtung eine Schuheinlage aufweist. In diesem Fall wird so ermöglicht, dass die Person eine Schuheinlage mit optimal eingestellten Werten für die Höhe und/oder die zumindest eine mechanische Eigenschaft weiterverwenden kann. Dies ist insbesondere von Vorteil, wenn die Schuheinlage bei der Einstellung in einem Schuh eingelegt ist, den die Person anschließend weiterverwenden möchte.

Die Offenbarung betriff zudem eine Schuhsimulationsvorrichtung, die dafür vorgesehen ist einen Fuß einer Person aufzunehmen. Die Schuhsimulationsvorrichtung verfügt über einen Schuhboden oder eine Schuheinlage, der/die eine Vielzahl von Auflageabschnitten aufweist, deren Höhe und/oder zumindest eine mechanische Eigenschaft jeweils einzeln steuerbar sind. Die Auflageabschnitte können eine beliebige Grundform aufweisen. In einer Ausführung bilden die einzelnen Auflageabschnitte ein Raster, bei dem alle Auflageabschnitte die gleiche Grundform aufweisen, beispielsweise Vierecke oder Kreise. In einer alternativen Ausführung weisen die Auflageabschnitte ergonomisch angepasste Formen auf, die einzelne Fußzonen nachbilden, beispielsweise eine elliptische Form für den Versenbereich oder eine Nierenform für den Ballen. Dadurch lässt sich eine bessere Anpassung der Werte für die mechanischen Eigenschaften der Auflageabschnitte an die jeweilige Funktion erzielen.

Vorzugsweise weist die Schuhsimulationsvorrichtung zumindest ein Haltemittel auf, das in der Länge verstellbar ist und dafür vorgesehen ist den Fuß der Person zu umfassen und auf dem Schuhboden zu fixieren. Damit lässt sich insbesondere auch der Oberbau des Schuhs simulieren und für die Person wahrnehmbar machen. In einer weiteren Ausführung der Erfindung weist zumindest ein Haltemittel jeweils zumindest ein Kontraktionselement aufweist, dessen Länge mittels einer Rechnereinheit und/oder durch eine Eingabevorrichtung und/oder manuell einstellbar ist.

In einer weiterführenden Ausführung der Erfindung weisen die Auflageabschnitte der Schuhsimulationsvorrichtung jeweils Sensoren auf, die dafür vorgesehen sind eine Kraft, die auf den jeweiligen Auflageabschnitt ausgeübt wird, zu bestimmen. Auf diese Weise ist es möglich die Schuhsimulationsvorrichtung selbst als Sensoreinheit zu verwenden, mit der man die Einstellung der Werte der Höhe und/oder der mechanischen Eigenschaften und/oder der Länge der Haltemittel überprüfen kann. Beispielsweise können Belastungsspitzen eines bestimmten Auflageabschnittes identifiziert werden, indem dieser eine gegenüber den umgebenden Auflageabschnitten überhöhte Kraft misst. So kann bereits beim Simulationsschritt, Begutachtungsschritt und/oder Optimierungsschritt eine automatische Überwachung der eingestellten Werte durchgeführt werden.

Ausführungsformen der Erfindung sind in den Figuren schematisch dargestellt. Es zeigen:
- Fig. 1:: eine Darstellung des erfindungsgemäßen Systems,
- Fig. 2:: eine Vorderansicht einer mit einem Fuß einer Person belasteten erfindungsgemäßen Schuhsimulationsvorrichtung,
- Fig. 3a:: eine Draufsicht einer mit einem Fuß der Person belasteten Schuhsimulationsvorrichtung mit einem Raster aus viereckigen Auflageabschnitten, und
- Fig. 3b:: eine Draufsicht einer mit einem Fuß einer Person belasteten Schuhsimulationsvorrichtung mit Auflageabschnitten, deren Form an ausgewählte Fußzonen angepasst ist.

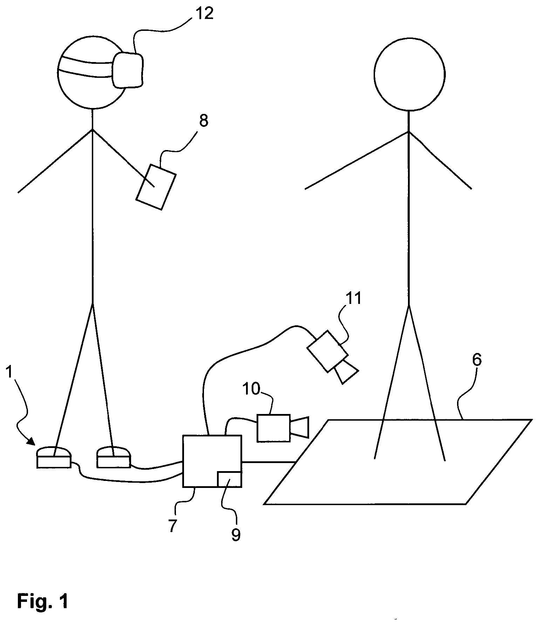

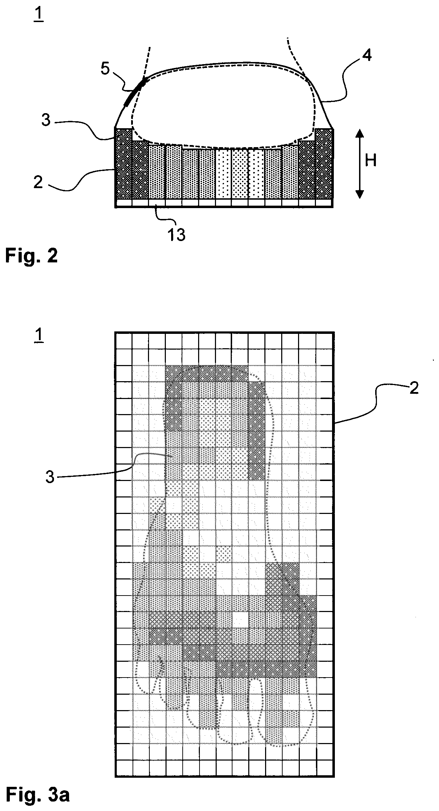

In Fig. 1 stellt ein System zur Simulation eines Schuhs dar, das zur Generierung von Daten für die Herstellung von individuell an eine Person angepassten Schuhen oder Schuheinlagen verwendbar ist. Das System umfasst eine Druckverteilungsmessvorrichtung 6, die dafür vorgesehen ist eine Druckverteilung zumindest einer Fußsohle der Person zu bestimmen, wobei Druckverteilungsmessvorrichtung 6 eine Druckmessplatte, eine Druckmessfolie oder eine sensitive Einlegesohle ist. Die gemessene Druckmessverteilung wird mittels einer Rechnereinheit 7 ausgewertet. Zudem weist das System eine Schuhsimulationsvorrichtung 1 auf, die dafür vorgesehen ist einen Schuh zu simulieren. Dafür weist die Schuhsimulationsvorrichtung 1 gemäß Fig. 2 einen Schuhboden 2 oder eine Schuheinlage mit einer Vielzahl von Auflageabschnitten 3 auf, deren Höhe H und/oder zumindest eine mechanische Eigenschaft auf Grundlage der bestimmten Druckverteilung mittels der Rechnereinheit 7 jeweils einzeln steuerbar sind.

Für die Bestimmung der räumlichen Form zumindest eines Fußes der Person weist das System bevorzugt zwei Scanvorrichtungen 10 und 11 auf. Damit lässt sich das Profil des Schuhbodens 2, beziehungsweise lassen sich die Höhen der einzelnen Auflageabschnitte 3, unmittelbar bestimmen. Vorzugsweise werden dafür optische 3D-Scanner eingesetzt. Die Scanvorrichtungen 10 und 11 sind zum Datenaustausch mit der Rechnereinheit über Kabel oder Funk verbunden.

Das System weist zudem eine Eingabevorrichtung 8 auf, die dafür vorgesehen ist die Höhe und/oder zumindest eine mechanische Eigenschaft der Auflageabschnitte 3 jeweils ortsabhängig einzustellen. Auf diese Weise kann die Person die Einstellungen der Simulationsvorrichtung 1 lokal optimieren, um eine wunschgemäße Eigenschaften zu realisieren.

Zudem verfügt das System über eine Anzeigeeinheit 12, die dafür vorgesehen ist bei der Person den Eindruck zu erwecken, dass dieser einen bestimmten Schuh trägt. Vorzugsweise handelt es sich dabei um eine Virtual-Reality-Brille 12, die die Person bei der Benutzung der Simulationsvorrichtung 1 trägt und die den Eindruck vermittelt, dass die Person einen bestimmten Schuh trägt. Der angezeigte Schuh kann ein Schuh eines bestimmten Typs oder bestimmten Designs sein. Insbesondere kann ein Schuh dargestellt werden, den die Person selbst ausgewählt oder gestaltet hat. Auf diese Weise lässt sich auch die äußere Erscheinung des Schuhs simulieren und individualisieren.

Die Rechnereinheit 7 weist eine Speichereinheit 9 auf, die dafür vorgesehen ist die eingestellten Werte für die die Höhe und/oder die zumindest eine mechanische Eigenschaft der Auflageabschnitte 3 und/oder die Länge eines Haltemittels 4 zu speichern. Insbesondere werden die von der Person als optimal oder wunschgemäß angesehenen Werte gespeichert. Diese Werte lassen sich dann verwenden, um eine bestimmte, insbesondere wunschgemäße, Einstellung zu reproduzieren. Insbesondere werden die abgespeicherten als optimal angesehenen Werte als Daten für die Herstellung eines individuell angepassten Schuhs verwendet, wobei der hergestellte Schuh die gleichen Eigenschaften aufweist wie der von der Simulationsvorrichtung 1 simulierte Schuh.

In einer nicht dargestellten Ausführung der Erfindung sind die Schuhsimulationsvorrichtungen 1 beweglich gelagert. Bevorzugt ist die Simulationsvorrichtung mobil ausgeführt, so dass die Person mit dieser herumgehen oder herumlaufen kann.

Fig. 2 zeigt eine schematische Darstellung der Schuhsimulationsvorrichtung 1 im benutzten, d.h. belasteten Zustand, wobei die Person auf dem Schuhboden 2 steht. Die Werte für die Höhe H der einzelnen Auflageabschnitte 3 sind so eingestellt, dass das Höhenprofil des Schuhbodens 2 der räumlichen Form der Fußsohle entspricht. Die unterschiedliche Textur der einzelnen Auflageabschnitte 3 deutet an, dass die Auflageelemente 3 jeweils unterschiedliche mechanische Eigenschaften aufweisen. Beispielsweise weisen die äußeren Auflageabschnitte 3 eine geringere Elastizität auf, um dem Fuß bei einer Belastung einen besseren Halt zu geben.

Die Schuhsimulationsvorrichtung 1 verfügt zumindest über ein Haltemittel 4, das dafür vorgesehen ist den Fuß der Person zu umfassen und auf dem Schuhboden 2 zu fixieren. Das Haltmittel ist beispielsweise ein flaches Band, welches an den Endpunkten am Schuhboden 2 befestigt ist. Die Länge des dargestellten Haltemittel 4 ist mittels eines Kontraktionselements 5 veränderbar. Die Länge wird mittels einer Rechnereinheit 7 und/oder durch eine Eingabevorrichtung 8 oder manuell eingestellt. Die Einstellung der Länge kann von der Person oder einem Dritten vorgenommen werden. Die Länge der Kontraktionseinheit 5 eines simulierten Schuhs wird bevorzugt in der Rechnereinheit 7 abgespeichert, so dass sich die Länge an einem anderen Ort zu einer andren Zeit reproduzieren lässt.

Zudem weisen die Auflageabschnitte 3 jeweils Sensoren 13 auf, die dafür vorgesehen sind eine Kraft, die auf den jeweiligen Auflageabschnitt 3 ausgeübt wird, zu messen. Somit verfügt die Schuhsimulationsvorrichtung 1 über eine Sensoreinheit, die es ermöglicht die eingestellten Werte im belasteten Zustand direkt zu überprüfen. Ungewünschte Kraftspitzen, respektive Druckspitzen, können damit vorteilhafterweise direkt registriert werden.

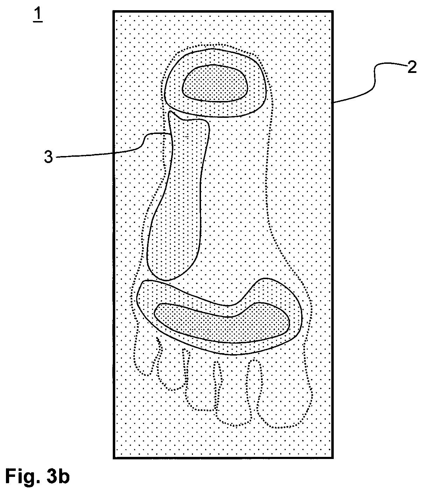

Die Auflageabschnitte 3 des Schuhbodens 2 der Schuhsimulationsvorrichtung 1 eine beliebige Form aufweisen. In Fig. 3a ist eine einer Ausführung dargestellt, bei der die einzelnen Auflageabschnitte 3 ein Raster bilden, bei dem alle Auflageabschnitte 3 die gleiche viereckige Grundform aufweisen. Der Fuß der Person ist dabei als gestrichelte Linie dargestellt. Durch die unterschiedliche Textur der einzelnen Auflageabschnitte 3 wird schematisch dargestellt, dass die einzelnen Auflageabschnitte unterschiedliche mechanische Eigenschaften aufweisen, die unabhängig voneinander einstellbar sind. Die äußeren Auflageabschnitte 3 weisen zur Stützung des Fußes eine geringere Elastizität auf. In Fig. 3b ist eine alternative Ausführung dargestellt, bei der die Auflageabschnitte 3 ergonomisch angepasste Formen aufweisen, die einzelne Fußzonen nachbilden. Beispielsweise haben die Auflageabschnitte 3 im Versenbereich eine elliptische Form und im Ballen Bereich eine Nierenform. Dadurch lässt sich eine bessere Anpassung der Werte für die mechanischen Eigenschaften der Auflageabschnitte 3 an die jeweilige Funktion erzielen.

Für das erfindungsgemäße Verfahren wird ferner eine Messfläche bzw. Messplatte verwendet, die eine Vielzahl dicht nebeneinander in einem Raster angeordneter Sensoren aufweist. Die Sensorflächen bzw. Sensorflächenabschnitte liegen in einer Fläche in x- und y-Richtung matrixförmig. Wird diese Messfläche von einem Schuh mit seiner Unterseite d. h. mit seiner Lauffläche (Schuhsohle) berührt, so werden eine Vielzahl von Drucksensoren berührt und einem mehr oder weniger starken Druck ausgesetzt. Hierbei formt jeder Drucksensor die physikalische Größe Druck in eine elektrische Größe (Spannung) um. Diese elektrischen Werte/Signale werden von einem Rechner erfasst und gespeichert. Hierbei stellt ein Rechnerprogramm die erfassten Werte grafisch und farbig dar, so dass zu erkennen ist, welche Stellen der Lauffläche des Schuhs einen stärkeren und welche einen schwächeren Druck auf die Messfläche ausüben.

Sowohl beim Stehen eines Benutzers mit seinem Schuh oder seinen Schuhen auf der Messfläche als auch beim Laufen oder Rennen auf der Messfläche werden wertvolle Messergebnisse in Form grafischer Bilder und Filme geschaffen, die zu den o. g. Wirkungen und Vorteilen führen.

Es sind die unterschiedlichsten Drucksensoren verwendbar. Sowohl passive als auch aktive Drucksensoren. Vorzugsweise werden piezoresistive und piezoelektrische Drucksensoren verwendet.

Die Messfläche/Messplatte ist vorzugsweise nicht quadratisch geformt, sondern weist eine größere Länge als Breite auf, so dass auf ihr in ihrer Längsrichtung gegangen/gelaufen werden kann. Hierbei ist die Messfläche eben zweidimensional. Alternativ ist die Messfläche dreidimensional gebogen/gewölbt, um eine natürliche Laufstrecke zu simulieren. Auch kann die Messfläche eine Ansteigung darstellen, in dem sie eine schräge Ebene bildet.

Die für die Beurteilung und Herstellung erforderlichen Daten werden noch dadurch verbessert, wenn die Daten/Messdaten eines bekannten optimalen Schuhs erfasst werden und diese Daten mit den durch eines der obigen Verfahren gemessenen Daten verglichen und verwertet werden.

## Patentansprüche

1. Verfahren zum Messen und Beurteilen der Funktionen und Form eines Schuhs insbesondere eines Sportschuhs wie eines Lauf- oder Skischuhs, wobei der Benutzer des Schuhs mit dem den Fuß bekleidenden Schuh ohne den Schuh mit Sensoren zu versehen auf einer Messfläche steht und/oder über eine Messfläche geht oder läuft, die eine Vielzahl von Drucksensoren aufweist, deren vom vermessenen Schuh erfasste Druckwerte von einem Rechnerprogramm ausgewertet werden, und dass nach den erfassten Druckwerten die Form, die Abmessungen und/oder die Materialien des Schuhbodens bestimmt werden und anhand der generierten Daten ein Schuh hergestellt wird.

2. Verfahren nach Anspruch 1, **dadurch gekennzeichnet, dass** nach den erfassten Druckwerten die Form und die Abmessungen des Schuhschaftes bestimmt werden.

3. Verfahren nach einem der vorherigen Ansprüche, umfassend die folgenden Schritte:
- einen Messschritt, bei dem eine Druckverteilung zumindest einer Fußsohle der Person mittels einer Druckverteilungsmessvorrichtung (6) auf einer Messfläche gemessen wird,
- einen Einstellschritt, bei dem eine Schuhsimulationsvorrichtung (1), die einen Schuhboden (2) oder eine Schuheinlage mit einer Vielzahl von Auflageabschnitten (3) aufweist, deren Höhe und/oder zumindest eine mechanische Eigenschaft jeweils einzeln steuerbar sind, lokal eingestellt wird, indem die Höhe und/oder die zumindest eine mechanische Eigenschaft der Auflageabschnitte (3) jeweils auf Werte eingestellt werden, die auf Grundlage der gemessenen Druckverteilung von einer Rechnereinheit (7) bestimmt werden, und
- einen Simulationsschritt, bei dem die Person den Schuhboden (2) belastet, um die Eigenschaften eines Schuhs wahrzunehmen, der die eingestellten Werte aufweist.

4. Verfahren nach Anspruch 3, **dadurch gekennzeichnet, dass** dieses einen Begutachtungsschritt aufweist, bei dem von der Person begutachtet wird, ob die lokal eingestellten Werte der Schuhsimulationsvorrichtung (1) dem persönlichen Optimum entsprechen oder, ob ein weiterer Einstellschritt mit geänderten eingestellten Werten erforderlich ist.

5. Verfahren nach Anspruch 3 oder 4, **dadurch gekennzeichnet, dass** dieses einen Optimierungsschritt umfasst, bei dem die Höhe und/oder die zumindest eine mechanische Eigenschaft der Auflageabschnitte (3) mittels einer Eingabevorrichtung (8) jeweils lokal eingestellt werden.

6. Verfahren nach einem der vorherigen Ansprüche, **dadurch gekennzeichnet, dass** zumindest ein Haltemittel (4), das die Simulationsvorrichtung (1) zur Fixierung des Fußes auf dem Schuhboden (2) aufweist, in seiner Länge verändert wird, vorzugsweise mittels einer Kontraktionseinheit (5).

7. Verfahren nach einem der vorherigen Ansprüche, **dadurch gekennzeichnet, dass** die von der Person als optimal angesehenen Werte für die Höhe und/oder die zumindest eine mechanische Eigenschaft der Auflageabschnitte (3) und/oder die Länge der Haltemittel (4) in einer Speichereinheit (9) abgespeichert werden.

8. Verfahren nach Anspruch 7, **dadurch gekennzeichnet, dass** die abgespeicherten Werte als Daten für die Herstellung von individuell angepassten Schuhen verwendet werden.

9. Verfahren nach einem der vorherigen Ansprüche, **dadurch gekennzeichnet, dass** beim Messschritt zusätzlich die räumliche Form des Fußes der Person mittels mindestens einer Scanvorrichtung (10, 11) bestimmt wird.

10. Verfahren nach einem der vorherigen Ansprüche, **dadurch gekennzeichnet, dass** der Person beim Benutzen der Schuhsimulationsvorrichtung (1) mittels einer Anzeigeeinheit (12) optisch suggeriert wird, dass sie einen Schuh eines bestimmten Typs oder eines bestimmten Designs, insbesondere eines selbst erzeugten Designs, trägt.

11. Verfahren nach einem der vorherigen Ansprüche, **dadurch gekennzeichnet, dass** die Daten/Messdaten eines bekannten optimalen Schuhs erfasst werden und diese Daten mit den durch eines der obigen Verfahren gemessenen Daten verglichen und verwertet werden.

12. System zur Simulation eines Schuhs zur Generierung von Daten für die Herstellung eines individuell an eine Person angepassten Schuhs unter Verwendung eines der obengenannten Verfahren, umfassend
- eine Druckmessplatte (6), mit einer Ortsauflösung und Drucksensitivität, mit der eine Druckverteilung eines Schuhs sowohl statisch als auch dynamisch beim Gehen oder Laufen bestimmbar ist,
- eine Rechnereinheit (7), die dafür vorgesehen ist die bestimmte Druckverteilung auszuwerten, und
- eine Schuhsimulationsvorrichtung (1), die einen Schuhboden (2) oder eine Schuheinlage mit einer Vielzahl von Auflageabschnitten (3) aufweist, deren Höhe und/oder zumindest eine mechanische Eigenschaft auf Grundlage der bestimmten Druckverteilung mittels der Rechnereinheit (7) jeweils einzeln steuerbar sind.

13. System nach Anspruch 12, **dadurch gekennzeichnet, dass** es zumindest eine Scanvorrichtung (10, 11) aufweist, die dafür vorgesehen ist die räumliche Form des Fußes zu bestimmen.

14. System nach einem der Ansprüche 12 bis 13, **dadurch gekennzeichnet, dass** es zumindest eine Eingabevorrichtung (8) aufweist, die dafür vorgesehen ist die Höhe und/oder zumindest eine mechanische Eigenschaft der Auflageabschnitte (3) jeweils ortsabhängig einzustellen.

15. System nach einem der Ansprüche 12 bis 14, **dadurch gekennzeichnet, dass** es zumindest eine Anzeigeeinheit (12) aufweist, die dafür vorgesehen ist bei der Person den Eindruck zu erwecken, dass dieser einen bestimmten Schuh trägt.

16. System nach einem der Ansprüche 12 bis 15, **dadurch gekennzeichnet, dass** die Schuhsimulationsvorrichtung (1) beweglich gelagert ist.

17. System nach einem der Ansprüche 12 bis 16, **dadurch gekennzeichnet, dass** die Schuhsimulationsvorrichtung (1), insbesondere die Schuheinlage (2), vom System trennbar ist und die eingestellten Werte für die Höhe und/oder die zumindest eine mechanische Eigenschaft beibehalten bleiben.

## Claims

1. Method for measuring and assessing the functions and shape of a shoe, in particular a sports shoe such as a running shoe or ski boot, wherein the user of the shoe, while wearing the shoe on the foot without the shoe being provided with sensors, stands on a measuring surface and/or walks or runs over a measuring surface, said measuring surface comprising a plurality of pressure sensors whose pressure values recorded in relation to the measured shoe are evaluated by a computer program, and wherein the shape, the dimensions and/or the materials of the bottom part of the shoe are determined according to the recorded pressure values, and a shoe is produced on the basis of the generated data.

2. Method according to Claim **1, characterized in that** the shape and dimensions of the upper part of the shoe are determined according to the recorded pressure values.

3. Method according to either of the preceding claims, comprising the following steps:
- a measurement step in which a pressure distribution of at least one sole of the person is measured on a measuring surface by means of a pressure distribution measuring apparatus (6),
- a setting step in which a shoe simulation apparatus (1) comprising a bottom part of the shoe (2) or an insole having a plurality of support portions (3), the height and/or at least one mechanical property of which can in each case be individually controlled, is set locally by in each case setting the height and/or the at least one mechanical property of the support portions (3) to values that are determined by a computer unit (7) on the basis of the measured pressure distribution, and
- a simulation step in which the person puts weight on the bottom part of the shoe (2) in order to perceive the characteristics of a shoe comprising the set values.

4. Method according to Claim 3, **characterized in that** it includes an assessment step in which the person reports whether the locally set values of the shoe simulation apparatus (1) correspond to their personal optimum, or whether a further setting step with modified set values is required.

5. Method according to Claim 3 or **4, characterized in that** it includes an optimization step in which the height and/or the at least one mechanical property of the support portions (3) are in each case set locally by means of an input apparatus (8).

6. Method according to any of the preceding claims, **characterized in that** at least one holding means (4), which comprises the simulation apparatus (1) for the purpose of securing the foot to the bottom part of the shoe (2), is changed in terms of its length, preferably by means of a contraction unit (5).

7. Method according to any of the preceding claims, **characterized in that** the values for the height and/or the at least one mechanical property of the support portions (3) and/or the length of the holding means (4) considered by the person as optimal are stored in a memory unit (9).

8. Method according to Claim 7, **characterized in that** the stored values are used as data for the production of individually adapted shoes.

9. Method according to any of the preceding claims, **characterized in that** during the measurement step the spatial shape of the foot of the person is additionally determined by means of at least one scanning apparatus (10, 11).

10. Method according to any of the preceding claims, **characterized in that** a display unit (12) of the shoe simulation apparatus (1) in use by the person is used to optically suggest to said person that they wear a shoe of a certain type or a certain design, in particular a self-generated design.

11. Method according to any of the preceding claims, **characterized in that** the data/measurement data of a known optimal shoe are recorded and said data are compared and utilized with the data measured by one of the above methods.

12. System for simulating a shoe for generating data for the production of a shoe individually adapted to a person using one of the above methods, comprising
- a pressure plate (6), having a spatial resolution and pressure sensitivity, with which a pressure distribution of a shoe can be determined both statically and dynamically when walking or running,
- a computer unit (7) that is provided to evaluate the determined pressure distribution, and
- a shoe simulation apparatus (1) comprising a bottom part of a shoe (2) or an insole having a plurality of support portions (3), the height and/or at least one mechanical property of which can in each case be individually controlled by the computer unit (7) on the basis of the determined pressure distribution.

13. System according to Claim 12, **characterized in that** it comprises at least one scanning apparatus (10, 11) that is provided to determine the spatial shape of the foot.

14. System according to either of Claims 12 and 13, **characterized in that** it comprises at least one input apparatus (8) that is provided to set, in each case in spatially dependent fashion, the height and/or at least one mechanical property of the support portions (3).

15. System according to any of Claims 12 to 14, **characterized in that** it comprises at least one display unit (12) that is provided to give the person the impression that they are wearing a certain shoe.

16. System according to any of Claims 12 to 15, **characterized in that** the shoe simulation apparatus (1) is movably mounted.

17. System according to any of Claims 12 to 16, **characterized in that** the shoe simulation apparatus (1), in particular the insole (2), is separable from the system, and the set values for the height and/or the at least one mechanical property are maintained.

## Revendications

1. Procédé de mesure et d'évaluation des fonctions et de la forme d'une chaussure, notamment d'une chaussure de sport telle qu'une chaussure de course ou de ski, l'utilisateur de la chaussure se tenant debout, avec la chaussure habillant le pied, sans équiper la chaussure de capteurs, sur une surface de mesure et/ou marche ou courant sur une surface de mesure qui présente une pluralité de capteurs de pression dont les valeurs de pression détectées par la chaussure mesurée sont évaluées par un programme informatique, et en ce qu'après les valeurs de pression détectées, la forme, les dimensions et/ou les matériaux de la semelle de la chaussure sont déterminés et une chaussure est fabriquée à partir des données générées.

2. Procédé selon la revendication 1, **caractérisé en ce qu'**après les valeurs de pression détectées, la forme et les dimensions de la tige de la chaussure sont déterminées.

3. Procédé selon l'une quelconque des revendications précédentes, comprenant les étapes suivantes :
- une étape de mesure, au cours de laquelle une répartition de pression d'au moins une plante de pied de la personne est mesurée sur une surface de mesure au moyen d'un dispositif de mesure de répartition de pression (6),
- une étape de réglage, au cours de laquelle un dispositif de simulation de chaussure (1), qui comporte une semelle de chaussure (2) ou une semelle intérieure de chaussure présentant une pluralité de sections d'appui (3) dont la hauteur et/ou au moins une propriété mécanique peuvent respectivement être commandées individuellement, est réglé localement en réglant la hauteur et/ou l'au moins une propriété mécanique des sections d'appui (3) à des valeurs déterminées par une unité de calcul (7) sur la base de la répartition de pression mesurée, et
- une étape de simulation, au cours de laquelle la personne sollicite la semelle de chaussure (2) afin de percevoir les caractéristiques d'une chaussure présentant les valeurs réglées.

4. Procédé selon la revendication 3, **caractérisé en ce qu'**il comprend une étape d'évaluation au cours de laquelle la personne évalue si les valeurs réglées localement du dispositif de simulation de chaussure (1) correspondent à l'optimum personnel ou si une autre étape de réglage avec des valeurs réglées modifiées est nécessaire.

5. Procédé selon la revendication 3 ou 4, **caractérisé en ce qu'**il comprend une étape d'optimisation au cours de laquelle la hauteur et/ou l'au moins une propriété mécanique des sections d'appui (3) sont réglées localement au moyen d'un dispositif de saisie (8).

6. Procédé selon l'une quelconque des revendications précédentes, **caractérisé en ce qu'**au moins un moyen de maintien (4), que comporte le dispositif de simulation (1) pour la fixation du pied sur la semelle de chaussure (2), est modifié en longueur, de préférence au moyen d'une unité de contraction (5).

7. Procédé selon l'une quelconque des revendications précédentes, **caractérisé en ce que** les valeurs considérées comme optimales par la personne pour la hauteur et/ou l'au moins une propriété mécanique des sections d'appui (3) et/ou la longueur des moyens de maintien (4) sont stockées dans une unité de stockage (9) .

8. Procédé selon la revendication 7, **caractérisé en ce que** les valeurs stockées sont utilisées comme données pour la fabrication de chaussures adaptées individuellement.

9. Procédé selon l'une quelconque des revendications précédentes, **caractérisé en ce que**, lors de l'étape de mesure, la forme spatiale du pied de la personne est également déterminée au moyen d'au moins un dispositif de balayage (10, 11).

10. Procédé selon l'une quelconque des revendications précédentes, **caractérisé en ce que**, lors de l'utilisation du dispositif de simulation de chaussure (1), il est suggéré optiquement à la personne, au moyen d'une unité d'affichage (12), qu'elle porte une chaussure d'un type ou d'une conception spécifique, notamment une conception qu'elle a elle-même créée.

11. Procédé selon l'une quelconque des revendications précédentes, **caractérisé en ce que** les données/mesures d'une chaussure optimale connue sont collectées et **en ce que** ces données sont comparées et évaluées avec les données mesurées par l'un des procédés ci-dessus.

12. Système de simulation d'une chaussure permettant de générer des données pour la fabrication d'une chaussure adaptée individuellement à une personne par utilisation de l'un des procédés mentionnés ci-dessus, comprenant
- une plaque de mesure de pression (6), présentant une certaine résolution spatiale et une sensibilité à la pression, permettant de déterminer une répartition de pression d'une chaussure de manière tant statique que dynamique en situation de marche ou de course,
- une unité de calcul (7) qui est prévue pour évaluer la répartition de pression déterminée, et
- un dispositif de simulation de chaussure (1), qui comporte une semelle de chaussure (2) ou une semelle intérieure de chaussure présentant une pluralité de sections d'appui (3) dont la hauteur et/ou au moins une propriété mécanique peuvent respectivement être commandées individuellement sur la base de la répartition de pression déterminée au moyen de l'unité de calcul (7).

13. Système selon la revendication 12, **caractérisé en ce qu'**il comporte au moins un dispositif de balayage (10, 11) qui est prévu pour déterminer la forme spatiale du pied.

14. Système selon l'une quelconque des revendications 12 à 13, **caractérisé en ce qu'**il comporte au moins un dispositif de saisie (8) qui est prévu pour régler la hauteur et/ou au moins une propriété mécanique des sections d'appui (3) en fonction de la position.

15. Système selon l'une quelconque des revendications 12 à 14, **caractérisé en ce qu'**il comporte au moins une unité d'affichage (12) qui est prévue pour donner à la personne l'impression qu'elle porte une chaussure spécifique.

16. Système selon l'une quelconque des revendications 12 à 15, **caractérisé en ce que** le dispositif de simulation de chaussure (1) est monté de manière mobile.

17. Système selon l'une quelconque des revendications 12 à 16, **caractérisé en ce que** le dispositif de simulation de chaussure (1), en particulier la semelle intérieure de chaussure (2), peut être séparé du système et **en ce que** les valeurs réglées pour la hauteur et/ou l'au moins une propriété mécanique restent inchangées.
